# FASCICULE DE BREVET EUROPEEN

(11) **EP 0 583 564 B1**
(45) Date de publication et mention de la délivrance du brevet: **09.10.1996**
(21) Numéro de dépôt: 93109262.1
(22) Date de dépôt: 09.06.1993
(51) Int. Cl.: C07C 67/293, C07C 69/738, C07D 303/40, C07C 69/95

(54) **Procédé pour la préparation de 3-oxo-2-pentyl-1-cyclopentène acétates d'alkyle**
Verfahren zur Herstellung von 3-Oxo-2-pentyl-1-cyclopentenessigsäurealkylester
Process for the preparation of 3-oxo-2-pentyl-1-cyclopentene alkyl acetates

(30) Priorité: 20.07.1992 CH 2279/92
(43) Date de publication de la demande: 23.02.1994
(73) Titulaire: FIRMENICH SA, CH-1211 Genève 8 (CH)
(72) Inventeur: Winter, Béat, Dr., CH-1233 Sezenove/Bernex (CH)
(74) Mandataire: Salvadori, Giuseppe, Dr.

(56) Documents cités:
- DE-B- 2 008 878
- CHEM. BER. vol. 112, 1979, pages 2256 - 2277 N. BENSEL ET AL

## Description

La présente invention a trait au domaine de la synthèse organique. En particulier, elle concerne un procédé pour la préparation de 3-oxo-2-pentyl-1-cylopentène acétates d'alkyle, composés utiles à titre d'intermédiaires pour la préparation de dérivés jasmoniques dotés de propriétés odorantes appréciées. En effet, il est connu que l'hydrogénation catalytique du 3-oxo-2-pentyl-1-cyclopentène acétate de méthyle, par exemple, à l'aide de Pd/C en présence de méthylate d'aluminium [DE-OS 2'162'820] conduit à la formation du cis-dihydrojasmonate de méthyle dont la note florale a suscité l'intérêt des parfumeurs.

L'art antérieur fait état de nombreuses voies de synthèse du 3-oxo-2-pentyl-1-cyclopentène acetate de méthyle. L'une seule d'entre elles a recours à un dérivé acyclique dans sa dernière étape [JP 58 118'536; Chem. Abstr. 1983, 99, 175481 d], les autres ayant comme intermédiaires clés des précurseurs cycliques. Suivant la nature de ces précurseurs les synthèses connues peuvent se classer en quatre catégories distinctes :
a. via une lactone gamma, par réarrangement avec l'acide polyphosphorique [NL 69 18' 228; Chem. Abstr. 1971, 75, 109 953 d];
b. via le dihydrojasmonate de méthyle par bromination et dehydrobromination [NL 70 02'279; Chem. Abstr. 1972, 76, 3462 g] ou par oxydation anodique du dérivé enol-acétate [J. Am. Chem. Soc. 1975, 97, 6144];
c. via la 2-pentyl-1,3-cyclopentanedione par réaction avec le malonate de diméthyle [voir par ex. DE-OS 2 008 833; Chem. Abstr. 1970, 73, 109 363 d];
d. via la 2-pentyl-2-cyclopentén-1-one par addition radicalaire d'hydroxyacétate de méthyle, suivie de deshydratation [Parf. Cosm. Sav. France 1972, 2 (8), 356]; par réaction avec le diazoacétate de méthyle [Perfum. Essent. Oil Rec. 1969, 267; JP 70 00862], ou par addition du bromoacétate de méthyle [J. Org. Chem. 1974, 39, 2637] ou du lithioacétate de méthyle suivie d'oxydation par l'acide chromique.

Aucune des synthèses mentionnées, pour la plupart de nature académique, n'a trouvé à ce jour une application industrielle, à grande échelle.

Nous avons maintenant découvert qu'il était possible de synthétiser de manière aisée et efficace des 3-oxo-2-pentyl-1-cyclopentène acétates d'alkyle par une réaction d'isomérisation, à l'aide d'un agent d'isomérisation acide, d'époxy-esters de formule 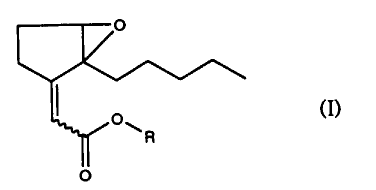 dans laquelle R sert à désigner un radical alkyle inférieur C₁-C₆, linéaire ou ramifié, et la ligne ondulée définit une liaison C-C de configuration cis ou trans.

L'invention a donc pour objet ledit procédé lequel est défini comme décrit par les revendications.

A titre d'agent d'isomérisation acide on peut employer un acide protique minéral ou organique ou un acide du type dit de Lewis. Parmi les acides actifs on peut mentionner à titre d'exemple les acides chlorhydrique, sulfurique, phosphorique, p-toluènesulfonique ou encore le BF_{3,} le SnCl₄ ou le TiCl₄. Des résines acides échangeuses d'ions peuvent également convenir.

L'isomérisation a lieu dans des conditions de réaction douces, ce qui rend le procédé particulièrement attrayant au point de vue industriel. En effet, des températures de l'ordre de 5-30°C se sont révélées parfaitement satisfaisantes pour l'obtention du produit désiré avec des rendements excellents, égals ou supérieurs à 90% en poids.

Les époxy-esters de formule (I), utilisés comme produits de départ dans le procédé de l'invention, sont des composés de structure nouvelle et à ce titre ils constituent également un des objets de la présente invention.

Ils peuvent être obtenus à partir de la 2,3-époxy-2-pentyl-1-cyclopentanone, composé décrit dans la littérature [voir par ex. Synthesis, 1975, 104], par un procédé caractérisé par une réaction du type dit de Wittig, plus précisement en appliquant la méthode élaborée par Wadsworth et Emmons, [voir J. Amer. Chem. Soc. 1961, 83, 1733] selon le schéma ci-après : 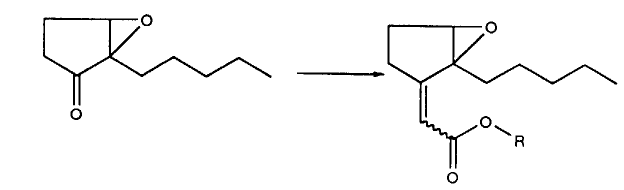 La réaction s'effectue par addition d'un dialkylphosphono-acétate d'alkyle, par exemple le diméthylphosphonoacétate de méthyle, ce qui permet l'obtention du 2,3-époxy-2-pentyl-1-cyclopentylidène acétate de méthyle. La réaction est conduite en analogie avec les méthodes décrites dans la littérature, les conditions particulières appliquées figurant ci-après.

L'invention est illustrée de manière plus détaillée à l'aide de l'exemple suivant dans lequel les températures sont indiquées en degrés centigrades et les abréviations ont le sens usuel dans l'art.

### Exemple

### Préparation de 3-oxo-2-pentyl-1-cyclopentène acétate de méthyle

### a) 2,3-Epoxy-2-pentyl-1-cyclopentanone

20 ml d'eau oxygénée à 70% dans l'eau (0,43 moles), suivis de 1,12 g (0,02 moles) d'hydroxyde de potassium en pastilles, ont été ajoutés à 10° sous agitation à une solution de 60,8 g (0,36 moles) de 3-oxo-2-pentyl-1-cyclopentène dans 100 ml de méthanol. Pendant l'addition la température augmente jusqu'à 25° et le mélange de réaction est maintenu à cette valeur de température pendant 1,5 h. Une nouvelle fraction d'égal volume d'eau oxygénée, respectivement KOH, a été ajoutée en deux portions au mélange réactionnel, ce qui conduit le taux de conversion à env. 75% après 4 heures de réaction.

10 g (0,07 moles) de K₂CO₃ ont été ensuite additionnés en deux portions au mélange obtenu en 2 h et le mélange a été maintenu sous agitation pendant deux heures supplémentaires à 25°. On a procédé ensuite à une extraction avec du CH₂Cl₂ et les extraits organiques combinés ont été lavés avec de l'eau, séchés sur du Na₂SO₄ et concentrés pour fournir un résidu qui, par distillation sous pression réduite, a donné 41 g (rend. 61%) de l'époxy-cétone désirée sous forme d'un liquide incolore. Eb.73°/66 Pa

### b) 2,3-Epoxy-2-pentyl-1-cyclopentylidène acétate de méthyle (par analogie avec Synthesis 1983, 284)

A une suspension d'hydrure de sodium (7,3 g ; 0,15 moles d'une dispersion à 50% dans l'huile), préalablement lavé avec de l'éther de pétrole 30-50, dans 200 ml de tétrahydrofurane, on a ajouté goutte à goutte à 10-20° 27,6 g (0,15 moles) de triméthylphosphonoacétate dans 200 ml de tétrahydrofurane (THF). 15 minutes après la fin de l'addition, on a ajouté au mélange de réaction, à 11-15°, une solution de 10,2 g (0.056 moles) de l'époxy-cétone préparée suivant la lettre (a) ci-dessus dans 150 ml de THF. Le mélange a été maintenu sous agitation pendant 16 h à 25° puis il a été versé dans 500 ml d'eau et extrait à l'éther. Les extraits organiques combinés ont été lavés avec une solution aqueuse saturée de NaCl, séchés sur Na₂SO₄ et concentrés. Le résidu obtenu a été distillé pour fournir une fraction à eb. 92-103°/4 Pa de l'époxy-ester désiré (11,9 g ; rend. 93% ; rapport isomérique trans/cis 94:5).
IR(film) : 2930, 2850, 1715, 1655, 1430, 1340, 1300, 1190, 1170, 1150, 1020, 850 cm⁻¹
¹H-RMN(360MHz,CDCl₃) : 0,90(t, J=7, 3H) ; 1,32(m, 7H) ; 1,80(m,2H) ; 2,00(m,1H) ; 2,13(dd, J=8, 14, 1H) ; 2,38(m,1H) ; 3,22(dd, J=9, 18, 1H) ; 3,73(s, 3H) ; 5,95(s, 1H) δ ppm
¹³C-RMN(90,5MHz,CDCl₃) : 162,9(s), 114,7(d), 100,7(s), 67,4(s), 65,0(d), 51,1(q), 31,9(t), 27,1(t), 27,0(t), 25,8(t), 24,7(t), 22,5(t), 13,9(q) δ ppm
SM : 224(M⁺,1), 208(2), 195(4), 167(100), 151(14), 139(24), 126(19), 107(20), 91(24), 79(40), 67(15), 55(20), 41(20).

### c) 3-oxo-2-pentyl-1-cyclopentène acétate de méthyle

0,1 ml (1,9 mmoles) d'acide sulfurique concentré a été ajouté sous agitation à 4° à une solution de l'époxy-ester préparé sous lettre (b) ci-dessus (8,6 g ; pureté 79%, 30 mmoles) dans 50 ml d'éther. Le mélange a été maintenu sous agitation tandis que la température augmentait graduellement jusqu'à env. 30°. Après avoir été maintenu à cette température pendant 1 h, le mélange a été dilué avec de l'éther, lavé avec une solution aqueuse saturée de NaHCO₃ et NaCl, puis séché sur Na₂SO₄ et concentré pour fournir 7,7 g de résidu. Ce dernier a donné par distillation dans un four à boules (0,04 mbar, temp.=110°) 6,97 g (pureté 93%; rend. 95%) de 3-oxo-2-pentyl-1-cyclopentène acétate de méthyle dont les caractères analytiques étaient les suivants :
IR(film) : 2950, 2920, 2850, 1735, 1695, 1635, 1430, 1350, 1255, 1190, 1170, 1110, 1055, 1015, 990 cm⁻¹
¹H-RMN(360MHz,CDCl₃) : 0,88(t, J=7, 3H) ; 1,2-1,4(m, 6H) ; 2,18(t, J=7, 2H) ; 2,41(m, 2H) ; 2,62(m, 2H) δ ppm
¹³C-RMN(90,5MHz,CDCl₃) : 209,2(s), 169,6(s), 163,5(s), 143,3(s), 52,3(q), 36,6(t), 34,4(t), 31,8(t), 29,7(t), 28,0(t), 23,2(t), 22,5(t), 13,0(q) δ ppm
SM : 224(M⁺, 0,5), 206(2), 168(14), 151(100), 135(12), 121(17), 109(55), 108(47), 93(33), 79(42), 67(13), 55(19), 41(28).

## Revendications

1. Procédé pour la préparation d'un 3-oxo-2-pentyl-1-cyclopentène acétate d'alkyle caractérisé en ce qu'on isomérise à l'aide d'un agent d'isomérisation acide un époxy-ester de formule 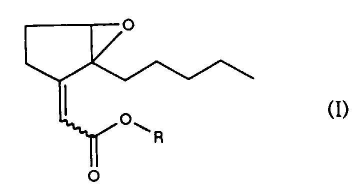 dans laquelle R sert à désigner un radical alkyle inférieur C₁-C₆, linéaire ou ramifié, et la ligne ondulée définit une liaison C-C de configuration cis ou trans.

2. Procédé suivant la revendication 1, caractérisé en ce qu'on utilise comme produit de départ de formule (I) le 2,3-époxy-2-pentyl-1-cyclopentylidène acétate de méthyle et qu'on obtient le 3-oxo-2-pentyl-1-cyclopentène acétate de méthyle.

3. Procédé suivant la revendication 1, caractérisé en ce que l'agent d'isomérisation acide est un acide protique minéral ou organique, un acide dit de Lewis ou une résine acide échangeuse d'ions.

4. Procédé suivant la revendication 3, caractérisé en ce qu'on utilise comme acide protique l'acide sulfurique.

5. Procédé selon la revendication 1, caractérisé en ce que la réaction d'isomérisation s'effectue à une température de 5 à 30°C.

6. Epoxy-ester de formule 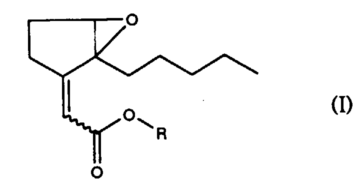 dans laquelle R sert à désigner un radical alkyle inférieur C₁-C₆, linéraire ou ramifié, et la ligne ondulée définit une liaison C-C de configuration cis ou trans.

7. A titre d'époxy-ester selon la revendication 6, le 2,3-époxy-2-pentyl-1-cyclopentylidène acétate de méthyle.

## Patentansprüche

1. Verfahren zur Herstellung eines 3-Oxo-2-pentyl-1-cyclopentenalkylacetats, dadurch gekennzeichnet, dass man einen Epoxyester der Formel 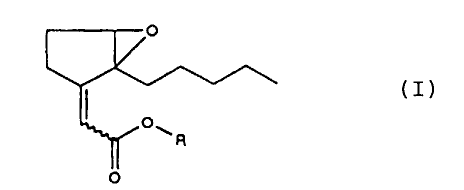 worin R für einen niederen, geradkettigen oder verzweigten C₁-C₆-Alkylrest steht und die Wellenlinie eine C-C-Bindung mit cis- oder trans-Konfiguration definiert, mit Hilfe eines saueren Isomerisierungsmittels isomerisiert.

2. Verfahren gemäss Patentanspruch 1, dadurch gekennzeichnet, dass man als Ausgangsverbindung der Formel (I) 2,3-Epoxy-2-pentyl-1-cyclopentyliden-methylacetat verwendet und man 3-Oxo-2-pentyl-1-cyclopenten-methylacetat erhält.

3. Verfahren gemäss Patentanspruch 1, dadurch gekennzeichnet, dass das sauere Isomerisierungsmittel eine anorganische oder organische Protonsäure ist, eine sogenannte Lewis-Säure oder ein saueres Ionenaustauschharz.

4. Verfahren gemäss Patentanspruch 3, dadurch gekennzeichnet, dass man als Protonsäure Schwefelsäure verwendet.

5. Verfahren gemäss Patentanspruch 1, dadurch gekennzeichnet, dass die Isomerisierungsreaktion bei einer Temperatur von 5 bis 30°C stattfindet.

6. Epoxyester der Formel 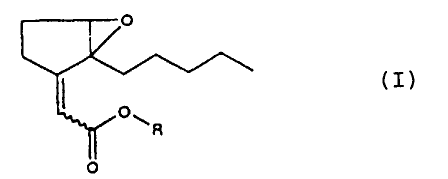 worin R für einen niederen, geradkettigen oder verzweigten C₁-C₆-Alkylrest steht und die Wellenlinie eine C-C-Bindung mit cis- oder trans-Konfiguration definiert.

7. 2,3-Epoxy-2-pentyl-1-cyclopentyliden-methylacetat als Epoxyester gemäss Patentanspruch 6.

## Claims

1. Process for the preparation of an alkyl 3-oxo-2-pentyl-1-cyclopentene acetate, characterized in that an epoxy-ester of formula 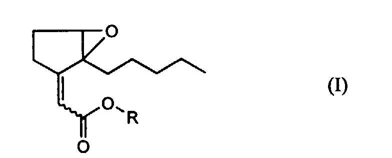 wherein R designates a lower linear or branched alkyl radical of C₁-C₆ and wherein the wavy line stands for a C-C bond of cis or trans configuration, is isomerized by means of an acidic isomerization agent.

2. Process according to claim 1, characterized in that one uses as starting product of formula(I) methyl 2,3-epoxy-2-pentyl-1-cyclopentylidene acetate and the product obtained is methyl 3-oxo-2-pentyl-1-cyclopentene acetate.

3. Process according to claim 1, characterized in that the acidic isomerization agent is a mineral or an organic protic acid, a Lewis type acid, or an acidic ion exchange resin.

4. Process according to claim 3, characterized in that sulfuric acid is used as the protic acid.

5. Process according to claim 1, characterized in that the isomerization reaction is carried out at a temperature of 5-30°C.

6. Epoxy-ester of formula 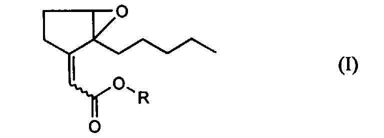 wherein R designates a lower linear or branched alkyl radical of C₁-C₆ and wherein the wavy line stands for a C-C bond of cis or trans configuration.

7. As epoxy-ester according to claim 6, methyl 2,3-epoxy-2-pentyl-1-cyclopentylidene acetate.
